# EUROPEAN PATENT APPLICATION

(11) **EP 0 650 076 A2**
(43) Date of publication of application: **26.04.1995**
(21) Application number: 94116406.3
(22) Date of filing: 18.10.1994
(51) Int. Cl.: G01T 1/164

(54) **Method and apparatus for retrofitting a multi-detector scintillation camera system to perform like a dual-head scintillation camera system of the type which is dedicated to cardiac spect studies**

(30) Priority: 25.10.1993 US 142155
(71) Applicant: SIEMENS MEDICAL SYSTEMS, INC., Iselin, New Jersey 08830 (US)
(72) Inventor: Greidanus, August, Schaumburg, IL 60194 (US)
(74) Representative: Fuchs, Franz-Josef, Dr.-Ing.

(57) **Abstract**

A triple-head scintillation camera system is retrofitted so as to perform like a dual-head system of the type which is dedicated to cardiac SPECT applications. This is done by collimating two of the detectors of the system with collimators that so collimate the collimated directions of view of the detectors as to cause the collimated directions of view to subtend a right angle.

## Description

### Background of the Invention

The invention relates to scintillation camera systems, and more particularly relates to multi-detector scintillation camera systems. In its most immediate sense, the invention relates to triple-head scintillation camera systems which are used to perform SPECT cardiac studies.

In a triple-detector (a so-called triple-head) scintillation camera system such as is marketed by Siemens Medical Systems, Inc. under the MULTISPECT 3 trademark, adjacent detectors have uncollimated directions of view which subtend an arc of 120 degrees. When such a camera is used to carry out a 180 degree cardiac SPECT study, the gantry must be rotated through 120 degrees to obtain sufficient data. Because of the geometry of the system and the anatomy of the patient, some of the data collected by one of the detectors and all of the data collected by another one of the detectors, is of little use. As a result, the performance of such a triple-head system in cardiac SPECT studies is only slightly better than the performance of a (much less expensive) single-head scintillation camera system.

Dual-head scintillation camera systems have been designed for dedicated use in cardiac SPECT applications. In such dedicated dual-head systems, the detectors have uncollimated directions of view which subtend an arc of 90 degrees. Such a system can acquire sufficient data for a cardiac SPECT study during a gantry rotation of only 90 degrees, and each of the detectors collects useful data at all positions during the study. This makes it possible to conduct a cardiac SPECT study in less time and increases patient throughput.

Only a few clinics can afford to have a special dual-head camera system whose utility is limited to cardiac SPECT applications. Furthermore, it is not presently feasible to retrofit or modify a triple-head scintillation camera system to orient the uncollimated directions of view of two of the detectors to subtend a right angle. Therefore, the great majority of nuclear medicine departments, and even departments which own conventional triple-head scintillation camera systems, are unable to obtain the advantages of dedicated dual-head scintillation camera systems when conducting cardiac SPECT studies.

It would be advantageous to provide method and apparatus which would be suitable for retrofitting a multi-head SPECT scintillation camera system, and particularly a triple-head SPECT scintillation camera system, so as to make the retrofitted system perform like a dedicated dual-head cardiac SPECT system.

One object of the invention is to provide method and apparatus which can be used to retrofit a multi-head SPECT scintillation camera system, and particularly a triple-head SPECT scintillation camera system, so that the retrofitted system performs like a dual-head dedicated cardiac SPECT system.

Another object is, in general, to improve on known scintillation camera systems of the multi-detector type.

In accordance with the invention, two collimators are secured to adjacent detectors in a multi-detector SPECT scintillation camera system. The collimators each have a view angle and a view offset angle, which are chosen to correspond to the detector geometry of the scintillation camera system on which the collimators are installed. The view offset angles are such that when the collimators are installed on the detectors, the collimated view angles of the detectors subtend an angle which is at least approximately a right angle or which, advantageously and in accordance with the preferred embodiment, is exactly a right angle. With such collimation, the two thus-collimated detectors mimic the performance of the two detectors in the above-described camera system which is dedicated to cardiac SPECT studies. In this way, a multi-head SPECT scintillation camera system, and advantageously a triple-head SPECT scintillation camera system, is retrofitted to perform like a dual-head scintillation camera system of the type which is dedicated to cardiac SPECT applications.

### Brief Description of the Drawings

The invention will be better understood with reference to the following illustrative and non-limiting drawings, in which:
Fig. 1 is a schematic drawing of a conventional triple-head scintillation camera system of the type used to perform SPECT studies;
Fig. 2 is a schematic drawing showing regions of data collection during a cardiac SPECT study and showing how a conventional triple-head scintillation camera system is used to perform such a study;
Fig. 3 is a schematic drawing of a known dual-head scintillation camera system which is intended for dedicated use in cardiac SPECT studies; and
Fig. 4 is a schematic drawing of the preferred embodiment of the invention.

### Detailed Description of Preferred Embodiments

The Figures are generally not to scale; they have been enlarged, schematized and simplified for clarity. Additionally, the same element is always indicated by the same reference numeral.

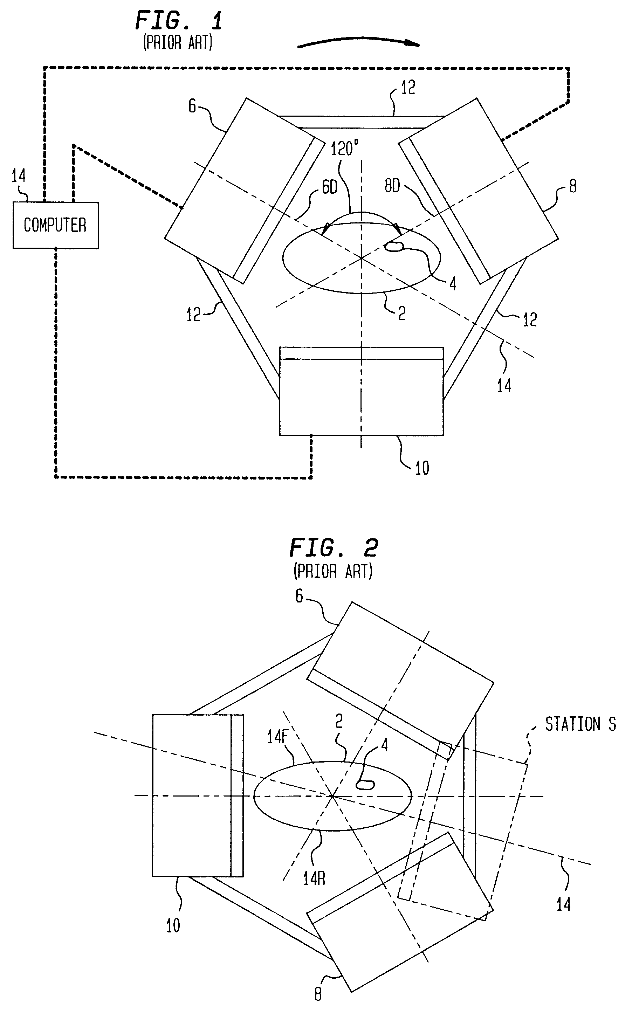

In a nuclear medicine cardiac SPECT study carried out in a triple-head scintillation camera system (see Fig. 1), a radioisotope (usually of thallium) is injected into the bloodstream of a patient 2 and collects in the patient's heart 4. Gamma radiation then leaves the patient 2 and is detected using detectors 6, 8 and 10. These detectors 6, 8 and 10 are supported by a gantry 12 and adjacent detectors (e.g. detectors 6 and 8) have uncollimated directions of view (e.g. 6D and 8D) which subtend an angle of 120 degrees. The gantry 12 rotates the detectors 6, 8 and 10 around the patient 2. Data generated by the detectors 6, 8 and 10 in response to incident gamma radiation is routed to a computer 14, which reconstructs a SPECT image of the heart 4.

In use, the detectors 6, 8 and 10 are always collimated; in the usual case, they are always collimated with parallel-hole collimators (so that their collimated directions of view are not changed from their uncollimated directions of view). For simplicity of illustration, the collimators for detectors 6, 8 and 10 are not shown in Fig. 1. Additionally, the gantry 12 of a conventional triple-head scintillation camera system permits the detectors 6, 8 and 10 to move radially inwardly and outwardly and does not, as the gantry 12 as illustrated seems to do, restrict the detectors 6, 8 and 10 to rotate within a predetermined circular orbit. The details of the gantry 12 have been eliminated for simplicity of illustration.

For cardiac SPECT applications, this known triple-head system configuration is not particularly advantageous. As can be seen in Fig. 2, the patient's body 2 can be treated as being divided by an axis 14 into two regions: the left anterior oblique region 14F and the right posterior oblique region 14R. Gamma radiation from the region 14F is quite useful, because the heart 4 is relatively close to the surface of the body 2 and the gamma radiation from the region 14F therefore has a relatively high signal-to-noise content. Gamma radiation from the region 14R, on the other hand, is less useful. This is because such radiation must pass through the patient's lungs, spine and posterior soft tissue and therefore is contaminated by a large quantity of noise by the time the radiation is picked up by a detector (such as detector 10).

For this reason, it is now generally accepted that a cardiac SPECT study can properly be carried out by sampling only the data from the region 14F. (Such a study is sometimes referred to as a 180 degree cardiac SPECT study.) To do such sampling completely (complete sampling is necessary to reduce artifacts in the reconstructed image), the camera gantry 12 must be rotated by 120 degrees (as shown in Fig. 2). With such rotation, the detector 8 does not collect useful information after it passes station S (where the axis 14 intersects the path of the detector 8 as it rotates around the patient 2). This is because after the detector 8 passes station S, the detector 8 is collecting non-useful data from the region 14R. Therefore, the detector 8 has little useful function for 60 degrees of rotation of the gantry 12. Additionally, the detector 10 is at all relevant times collecting nonuseful data from the region 14R. Consequently, for fully 1/2 of the rotation of the gantry 14, the triple-head scintillation camera system is collecting no data other than that which a much less expensive single-head scintillation camera system would collect.

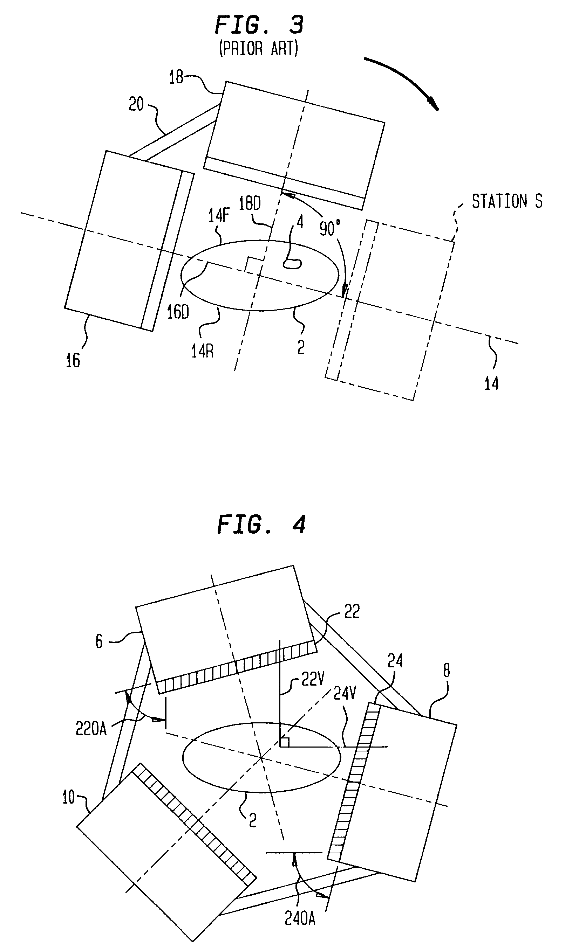

In a camera design (see Fig. 3) which is intended for dedicated use for cardiac SPECT studies, two detectors 16 and 18 are supported by a gantry 20 for rotation around a patient 2. The uncollimated directions of view 16D and 18D of the detectors 16 and 18 subtend an angle of 90 degrees. Such a camera design can sample all the data from the region 14F in only 90 degrees of rotation of the gantry 20 (see Fig. 3). Furthermore, when conducting the study, each detector 16 and 18 is collecting useful data at all the positions of the gantry 20. This camera design is therefore especially well suited to dedicated use in cardiac SPECT studies.

In accordance with a preferred embodiment of the invention intended for application to a triple-head SPECT scintillation camera system (see Fig. 4), each of two parallel-hole collimators 22 and 24 is mounted to a corresponding one of the detectors 6 and 8. Each of the collimators 22 and 24 has a view axis 22V and 24V respectively and a view axis offset angle ("slant angle") 22OA and 24OA respectively. The view axis offset angles 22OA and 24OA are so chosen (in this example, both angles equal 75 degrees, but such equality is not necessary to the practice of the invention) that when the collimators 22 and 24 are mounted to the detectors 6 and 8, the view axes 22V and 24V subtend an angle which is at least approximately a right angle and which advantageously is exactly a right angle.

When the detectors 6 and 8 are collimated using parallel hole collimators 22 and 24 in accordance with the preferred embodiment of the invention, the collimated directions of view of the detectors 6 and 8 lie along the view axes 22V and 24V of the collimators 22 and 24. Therefore, in the preferred embodiment of the invention, the collimated directions of view of the detectors 6 and 8 subtend an angle of 90 degrees, and the detectors 6 and 8 can completely sample all the data from the region 14F in 90 degrees of rotation of the camera gantry 12. In this way, a triple-head SPECT scintillation camera system which is retrofitted in accordance with the invention performs like a dual-head scintillation camera system of the type which is dedicated to cardiac SPECT applications.

It will be understood that the collimators 22 and 24 are not permanently secured to the detectors 6 and 8. This is because the triple-head scintillation camera system will generally be used for applications other than cardiac SPECT studies. The collimators 22 and 24 are detachably secured to the detectors 6 and 8 and may be replaced by other collimators for nuclear medicine studies which are not of the cardiac SPECT type.

The invention is not restricted to use on a triple-head scintillation camera system. The invention may be used on a camera system having any number of heads provided it is feasible to manufacture collimators having the necessary view axis offset angles. Furthermore, collimators in accordance with the invention need not necessarily be of the parallel-hole type, and while the view axes of the collimators advantageously subtend exactly 90 degrees, there can be some deviation from this without adversely affecting system performance.

Although a preferred embodiment has been described above, the scope of the invention is limited only by the following claims:

## Claims

1. Scintillation camera apparatus comprising:
first and second detectors having uncollimated directions of view which subtend an angle other than a right angle; and
first and second collimators, each being detachably securable to a corresponding one of the detectors, each collimator having a view axis and a view axis offset angle, the view offset angles being such as to cause the collimated directions of view of the detectors to subtend a right angle.

2. The apparatus of claim 1, wherein the first and second collimators are parallel hole collimators.

3. The apparatus of claim 1, wherein the uncollimated directions of view of the detectors subtend an angle of 120 degrees and the view axis offset angles of the collimators are 75 degrees.

4. A method of retrofitting a conventional three-detector scintillation camera system so that the retrofitted system has performance which during cardiac SPECT studies approximates performance attained using a dual-head scintillation camera system of the type which is dedicated to cardiac SPECT applications, comprising the step of securing, to two of the three detectors of the scintillation camera system, parallel hole collimators having view offset angles of 75 degrees.

5. A scintillation camera system comprising:
first and second detectors;
a gantry supporting the detectors such that they have uncollimated directions of view which subtend an angle that is other than a right angle;
first and second collimators, each being detachably securable to a corresponding one of the detectors, each collimator having a view axis and a view axis offset angle, the view offset angles being such as to cause the collimated directions of view of the detectors to subtend a right angle; and
means, connected to the detectors, for acquiring and processing information relating to scintillation events which occur therein.

6. Scintillation camera apparatus comprising:
first and second detectors having uncollimated directions of view which subtend an angle other than a right angle; and
first and second collimators, each secured to a corresponding one of the detectors, each collimator having a view axis and a view axis offset angle, the view offset angles being such as to cause the collimated directions of view of the detectors to subtend an angle which approximately equals a right angle.

7. Triple-head scintillation camera apparatus which is adapted for retrofitting into a scintillation camera system which performs like a dual-head scintillation camera system of the type which is dedicated to cardiac SPECT applications, comprising:
first, second and third detectors;
a gantry supporting the detectors such that adjacent detectors have uncollimated directions of view which subtend an angle of 120 degrees;
first and second collimators, each collimator being detachably securable to a corresponding one of the detectors and having a view axis and a view axis offset angle of 75 degrees, whereby the collimated directions of view of the detectors with the collimators secured thereto subtends a right angle; and
means, connected to the detectors, for acquiring and processing information relating to scintillation events which occur therein.
